(19) **Europäisches Patentamt** / **European Patent Office** / **Office européen des brevets**

(11) **EP 3 809 342 A1**

(12) **EUROPEAN PATENT APPLICATION**

(43) Date of publication:
21.04.2021 Bulletin 2021/16

(51) Int Cl.:
**G06N 20/20** (2019.01)

(21) Application number: 20200864.5

(22) Date of filing: 08.10.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.10.2019 US 201962915459 P**

(71) Applicant: **The Chinese University Of Hong Kong Hong Kong 999077 (CN)**

(72) Inventors:
• **WANG, Maggie Haitian New Territories, Hong Kong SAR 999077 (CN)**
• **XIA, Xiaoxuan Linqu, Shandong (CN)**
• **ZEE, Benny Chung-Ying Kowloon, Hong Kong (CN)**

(74) Representative: **Foster, Mark Charles et al Mathisen & Macara LLP Communications House South Street Staines-upon-Thames, Middlesex TW18 4PR (GB)**

(54) **PREDICTION MODELS INCORPORATING STRATIFICATION OF DATA**

(57) A Prism Vote process can be used to make predictions based on a data set that may be heterogeneous. The data set can be stratified, e.g., using techniques adapted from principal component analysis. A prediction model can be trained independently on each stratum of the data set. To make a prediction for a "testing" data sample, the prediction model for each stratum can be used to provide a per-stratum prediction of an outcome, and a per-stratum probability that the testing data sample belongs to each stratum can be determined. The predicted outcome (e.g., a probability of a particular outcome) can be computed from the per-stratum predictions weighted according to the per-stratum probabilities.

*FIG. 1*

**Description**

CROSS-REFERENCE TO RELATED APPLICATIONS

This application claims priority to U.S. Provisional Application No. 62/915,459, filed October 15, 2019.

BACKGROUND

This disclosure relates generally to prediction of outcomes and in particular to prediction models that incorporate stratification of data.

The ability to predict outcomes can be useful in many contexts. For example, in the field of medicine, optimal recommendations related to cancer screenings (e.g., how often to perform screening and/or which screening tests to perform) may vary depending on the particular patient's risk of cancer. As another example, if a patient has a particular disease, the choice of treatment may vary depending on the likelihood of a favorable outcome.

Traditionally, statistical correlations have been used to generate predictions based on one or more variables, using techniques such as linear or logistic regression. In traditional methods, a research team designs a study to test a specific hypothesis that a particular variable (or set of variables) correlates with a particular outcome, then collects a number of samples sufficient to test the hypothesis, with the number being determined in advance based on the expected effect size, potential confounding variables that are to be controlled for, and so on.

More recently, machine learning has increased the potential for individualized predictions, particularly when confronted with large numbers of potentially relevant variables. A machine-learning classifier is given a (usually large) set of "training" data that represent cases where a set of variables and an outcome are known. The classifier can be trained using known training procedures to optimize a (mathematically complex) formula that predicts outcomes from the variables. Very often, the training of a machine-learning classifier is a dynamic process, with new samples being added to the training data set as information about outcomes for more cases becomes available. Training of the classifier is repeated from time to time to take advantage of the additional information.

SUMMARY

As data sets become larger, they also tend to become more heterogeneous. This increasing heterogeneity can decrease the accuracy of prediction algorithms that are based on treating the entire training data set as a single population. For instance, a variable that may is strong predictor for one segment of the population may have little effect on another segment.

Certain embodiments of the claimed invention relate to techniques for making predictions based on a data set that may be heterogeneous. The heterogeneity is handled using a systematic approach that stratifies the data, e.g., using techniques adapted from principal component analysis. A prediction model can be trained independently on each stratum of the data set. To make a prediction for a "testing" data sample, the prediction model for each stratum can be used to provide a per-stratum prediction of an outcome, and a per-stratum probability that the testing data sample belongs to each stratum can be determined. The predicted outcome (e.g., a probability of a particular outcome) can be computed from the per-stratum predictions weighted according to the per-stratum probabilities.

The techniques described herein are applicable to any data set that may represent a heterogeneous population. While examples described herein relate to disease prediction using genomic data, similar techniques may be applied in other contexts. For example, in the field of health care, the data may include biomarkers other than genomic data (e.g., blood chemistry data; medical imaging data; biometric parameters such as heart rate or blood pressure; family medical history; behavioral parameters such as diet or exercise), and the prediction may relate to a diagnosis (e.g., presence or absence of a particular disease), likelihood of developing a disease, expected response to a particular course of treatment, and so on. The techniques described herein can also be applied to other fields, such as finance (e.g., predicting future investment returns or likelihood of default on a loan), insurance (e.g., predicting likely value of future claims by an insured person), and so on.

The following detailed description, together with the accompanying drawings, will provide a better understanding of the nature and advantages of the claimed invention.

BRIEF DESCRIPTION OF THE DRAWINGS

FIG. 1 shows a flow diagram of a process for predicting likelihood of an outcome according to an embodiment of the present invention.

FIG. 2 shows a flow diagram of a process for segmenting, or stratifying, a training set that can be used with the process of FIG. 1 in some embodiments of the present invention.

FIG. 3 shows a flow diagram of a process for computing a predicted outcome that can be used with the process of FIG. 1 in some embodiments of the present invention.

FIGs. 4A-4D show four graphs illustrating results of applying a process according to an embodiment of the present invention to simulated data sets.

FIG. 5 is a bar chart illustrating results of applying a process according to an embodiment of the present invention to simulated data sets.

FIG. 6 is a graph showing receiver operating characteristic (ROC) curves for Alzheimer's disease data analyzed using a process according to an embodiment of the present invention and a global logistic regression.

FIG. 7 is a graph showing ROC curves for schizophrenia data analyzed using a process according to an embodiment of the present invention and a global logistic regression.

DETAILED DESCRIPTION

To provide an understanding of various features of the claimed invention, embodiments are described in which genomic data is used to predict likelihood of an individual developing a specific disease. It should be understood, however, that the same techniques can be applied to other types of data, and the invention is not limited to genomic data, to disease prediction, or to the field of health care.

**Prism Vote Processes**

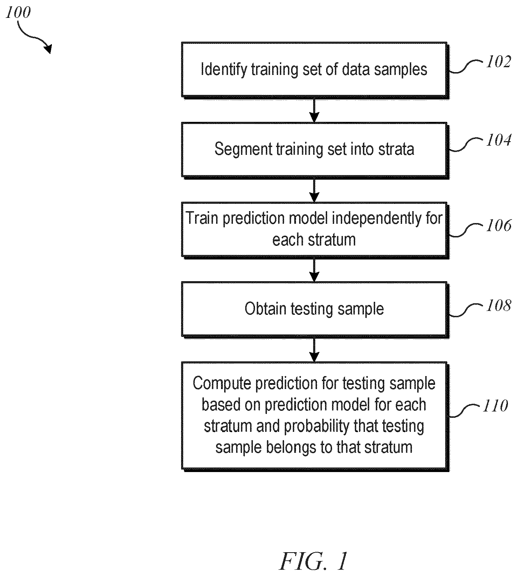

FIG. 1 shows a flow diagram of a process 100 for predicting likelihood of an outcome according to an embodiment of the present invention. Process 100 can be implemented using a computer system of suitable design.

At block 102, a training set of data samples is identified. The training set can include any number ($N$) of individual data samples, which can be samples corresponding to different individual subjects or different instances of a phenomenon being studied. For each data sample $x_i$, it is assumed that a set of $p$ variables $\{x_{ij}\}$ (for $j = 1,...,p$) has been measured and that an outcome $y_i$ is known. For example, the set of variables $\{x_{ij}\}$ can represent genomic data indicating presence or absence of each of $p$ different single nucleotide polymorphisms (SNPs). For each SNP, the variable $x_{ij}$ can have the value 0, 1, or 2, corresponding to the minor allele frequency count in the genotype observed for a subject. For example, if G is the minor allele and the observed genotype is GG, the SNP value is coded as 2. If the observed genotype is CC, the SNP value is coded as 0. In the case of disease prediction, outcome $y_i$ can indicate presence ($y_i = 1$) or absence ($y_i = 0$) of a disease. In cases such as predicting a variable physical characteristic (e.g., blood glucose level or cholesterol level), outcome $y_i$ can be a continuously-valued variable. Other coding schemes can be used, depending on the particular information being represented in the data samples $x_i$.

At block 104, the training set of data samples is segmented into a number of strata. The number of strata can be chosen based on the size of the data set (i.e., the number $N$ of samples) and a minimum average number of samples ($C$) for a stratum. In some embodiments, the number of strata ($K$) can be chosen within the range $2 \leq K \leq N/C$. As described below, a prediction model is trained for each stratum and, the minimum average number of samples $C$ can be determined based in part on the particular prediction model that is to be trained. For example, many prediction models based on linear regression assume a data set of a minimum size (which depends on the number of variables in the model), and an appropriate choice of $C$ may be 30, 50, 100 or the like, depending on the number of variables. Machine-learning classifiers may require even larger training sets to produce reliable prediction models, particularly if the number of variables is large. An example of a technique that can be used in some embodiments to optimize the number of strata for a given training data set is described below.

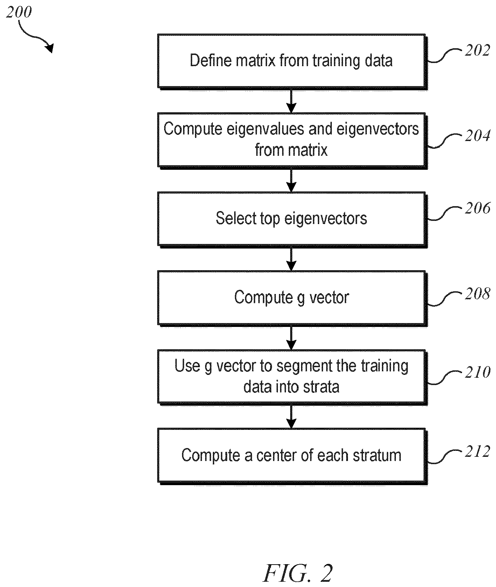

FIG. 2 shows a flow diagram of a process 200 for segmenting, or stratifying, a training set that can be implemented at block 104 of process 100. Process 200 involves using a matrix representation of the training data and elements of principal component analysis to define similarity.

At block 202, a matrix **X** is defined from the training data. In some embodiments, each row of matrix **X** can correspond to a data sample $x_i$ and each column to a variable. Thus, for a training data set of $N$ samples, each having $p$ variables, **X** can be an $N \times p$ matrix. Depending on the particular combination of variables, it may be desirable to perform a normalization operation on each column (e.g., normalizing each variable to a percentage of a maximum value) so that all variables are in a similar numerical range.

At block 204, eigenvalues and eigenvectors can be computed for the **X** matrix. Specifically, eigenvalues $\lambda_j$ and eigenvectors $\mathbf{v}_j$ (for $j = 1, \ldots N$) can be computed according to:

$$\mathbf{XX}'\mathbf{v}_j = \lambda_j \mathbf{v}_j , \qquad (1)$$

where **X' is** the matrix transpose **of X.** It is assumed that the eigenvalues are ordered by magnitude from largest to smallest.

At block 206, a subset consisting of a number $q$ of the eigenvectors $\mathbf{v}_j$ can be selected as the "top" (or most significant) eigenvectors. For example, the eigenvalues $\lambda_j$ can be ordered according to magnitude, and the $q$ largest eigenvalues can be identified as representing the most statistically significant components. The choice of $q$ in a given case can be determined using a scree plot or similar techniques.) The $q$ eigenvectors $\mathbf{v}_j$ corresponding to the $q$ most statistically significant components are selected as the top eigenvectors.

At block 208, a **g** vector can be computed from the top $q$ eigenvectors according to:

$$\mathbf{g} = \sum_{j=1}^{q} a_j \mathbf{v}_j , \qquad (2)$$

where

$$a_j = \frac{\lambda_j}{\sum_{i=1}^{q} \lambda_i} . \qquad (3)$$

The **g** vector is an $N$-component vector that summarizes the variation of the $N$ data samples measured in eigenvalues $\lambda_j$ along the top $q$ eigenvectors, and each component of **g** corresponds to a different data sample $\mathbf{x}_i$.

At block 210, the **g** vector can be used to segment the training data into $K$ strata. Specifically, the components of the **g** vector can be ordered by magnitude, and quantiles of **g** can be assigned to each stratum. For example, if $K = 2$, the data samples corresponding to the first $N/2$ components of (ordered) **g** can be assigned to one stratum and the rest to the other stratum. If K = 20, each quantile can include $N/20$ data samples. (Since there is an eigenvalue corresponding to each data sample, this also implies that parts of the eigenvectors are assigned to a stratum.)

In some embodiments, the same number of data samples (e.g., $N/K$) can be assigned to each stratum. If N/K is not an integer, rounding techniques can be used, with the result that the number of data samples in different strata may differ by 1. In other instances, the segmentation can be unequal, e.g., based on patterns in the eigenvalues or in the components of the **g** vector that suggest a natural clustering of the data samples. As long as each stratum includes at least the minimum number of data samples to support training of a prediction model on that stratum, different strata can include different numbers of data samples without restriction.

In cases where the variables $\{x_{ij}\}$ represent genomic variation (e.g., where the variables represent SNPs), the eigenvectors can be interpreted as an ancestry direction. Subjects with high variation among the top $q$ eigenvectors are genetically closer and are grouped together. In this context, process 200 can be understood as an approach to breaking out and integrating heritability as a spectrum. In other cases, the interpretation may be different, but the general approach is to form clustered cohorts within a set of data samples where the clustering reflects endogenous structure within the data set.

At block 212, a center of each stratum can be computed as the mean of the top $q$ eigenvectors in the stratum. Specifically, for a stratum $k$ (where $k = 1, \ldots, K$), a center can be defined as a $q$-component vector $\mathbf{c}^k$ having components

$$c_j^k = \frac{1}{n_k} \sum_{i=i_0}^{i_0+n_k} v_{ij} , \qquad (4)$$

where $i_0$ is an index corresponding to the first data sample in the stratum, $v_{ij}$ is the $i$th component of the $j$th eigenvector, and $j = 1, \ldots, q$. As described below, the vectors $\mathbf{c}^k$ can be used to determine a probability that a new data sample belongs to stratum $k$.

Referring again to FIG. 1, after the training data has been segmented into strata (also referred to as stratifying the training data), at block 106 a prediction model is trained independently for each stratum. In some embodiments, the same prediction model is used for all strata but because the training data sets are different, the models for different strata may yield different predictions.

By way of example, the prediction model can be a linear regression model that predicts an outcome as a continuously-valued variable $y$. A linear regression model for a training set of $N$ data samples having $p$ variables can be expressed as:

$$y = X\beta + \varepsilon, \tag{5}$$

where $y = \begin{pmatrix} y_1 \\ \vdots \\ y_N \end{pmatrix}$ is a vector of (known) outcomes, $X = \begin{pmatrix} 1 & x_{11} & \cdots & x_{1p} \\ 1 & x_{21} & \cdots & x_{2p} \\ \vdots & \vdots & \ddots & \vdots \\ 1 & x_{N1} & \cdots & x_{Np} \end{pmatrix}$ is a matrix of (known) obser-

vations, and $\beta = \begin{pmatrix} \beta_0 \\ \vdots \\ \beta_p \end{pmatrix}$ and $\varepsilon = \begin{pmatrix} \varepsilon_1 \\ \vdots \\ \varepsilon_N \end{pmatrix}$ are parameters to be computed. $\{\varepsilon_1, \varepsilon_2,...,\varepsilon_N\}$ are independent of each other, with mean 0 and variance $\sigma^2$. Techniques for computing the parameters of a linear regression model from training data are known in the art and may be applied in the context of process 100.

In accordance with block 106 of process 100, the linear regression model of Eq. (5) is applied to each stratum separately. That is, instead of a single model, there are $K$ models of the form:

$$y^k = X^k \beta^k + \varepsilon^k, \tag{6}$$

where $y^k = \begin{pmatrix} y_1^k \\ \vdots \\ y_{n_k}^k \end{pmatrix}$, $X^k = \begin{pmatrix} 1 & x_{11}^k & \cdots & x_{1p}^k \\ 1 & x_{21}^k & \cdots & x_{2p}^k \\ \vdots & \vdots & \ddots & \vdots \\ 1 & x_{n_k 1}^k & \cdots & x_{n_k p}^k \end{pmatrix}$, $\beta^k = \begin{pmatrix} \beta_0^k \\ \vdots \\ \beta_p^k \end{pmatrix}$, $\varepsilon^k = \begin{pmatrix} \varepsilon_1^k \\ \vdots \\ \varepsilon_{n_k}^k \end{pmatrix}$, and $k = 1, ..., K$.

The components of each $\varepsilon^k$ are independently and normally distributed, with mean 0 and variance $\sigma_0^2$ (in this example, variance is assumed to be the same for all $k$).

While linear regression is used as an example, it should be understood that process 100 is not limited to any specific prediction model. Other prediction models, such as logistic regression models, nonlinear models, support vector machine (SVM) models, deep learning models, can be used, provided that sufficient data is available to train the prediction model independently for each stratum. Depending on the particular prediction model, training can include computing a linear regression (e.g., using Eq. (6)), applying a machine-learning algorithm to train a deep-learning classifier, or any other technique for training a particular prediction model based on available training data.

The trained prediction models can be used to make predictions. For example, at block 108 of process 100, a "testing" sample ($s$) can be obtained. As used herein, the testing sample $s$ can be any data sample that was not used in training the prediction models and for which the relevant variables $\mathbf{x}_s = \{x_{sj}\}$ are known. In some instances, the outcome ($y_s$) for the testing sample $s$ is not known (e.g., when using the trained prediction models to predict an outcome for a patient in clinical practice). In other instance, the outcome $y_s$ may be known (e.g., when testing a trained prediction model to assess its performance).

At block 110, a predicted outcome for the testing sample can be computed based on the prediction model for each stratum and a probability that the testing sample belongs to that stratum. A per-stratum prediction can be computed from the prediction model for each stratum, and the per-stratum predictions can be combined using weights based on the probability that the testing sample belongs to each stratum.

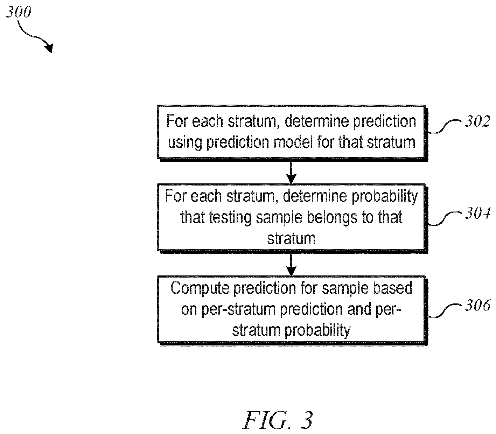

FIG. 3 shows a flow diagram of a process 300 for computing a predicted outcome that can be used at block 110. At block 302, for each stratum $k$, a per-stratum prediction ($y^k$) is computed on the assumption that the testing sample belongs to stratum $k$. For example, the (known) variables $\{x_{sj}\}$ associated with the testing sample $s$ can be provided as inputs to the trained prediction model for each stratum, and the prediction model can be applied to compute the predicted outcome.

At block 304, for each stratum $k$, a per-stratum probability that the testing sample $s$ belongs to the stratum can be determined. In some embodiments, the probability of a testing sample belonging to a particular stratum can be based on a distance to a center of that stratum and a Bayesian analysis.

For example, the center of a stratum can be defined according to Eq. (4) above. Distance to the center can be computed as follows. First, eigenvectors for a test set that includes test sample s are computed using a matrix **XX'** that includes test sample $s$. (The matrix **XX'** may also include some or all of the training samples.) Accordingly, an eigenvector $\mathbf{v}_s$ for test sample $s$ can be determined. The distance between $\mathbf{v}_s$ and the center $\mathbf{c}^k$ of stratum $k$ (defined according to Eq. (4) above) can be computed as:

$$d_s^k = (\mathbf{v}_s - \mathbf{c}^k)'(\mathbf{\Sigma}^k)^{-1}(\mathbf{v}_s - \mathbf{c}^k)$$

$$= \sum_{j=1}^{q} \frac{1}{\sigma_j^2(k)}\left(v_{sj} - c_j^k\right)^2 \sim \chi_q^2 , \tag{7}$$

where

$$\Sigma^k = \begin{pmatrix} \sigma_1^2(k) & \dots & 0 \\ \vdots & \ddots & \vdots \\ 0 & \dots & \sigma_q^2(k) \end{pmatrix}_{q \times q} \tag{8}$$

and $\sigma_j^2(k) = var(v_{ij})$, $i = 1, \dots, n_k$. The off-diagonal terms are 0 because the eigenvectors are orthogonal. The (tail) probability of observing a sample $s$ having variables $\mathbf{x}_s = \{x_{sj}\}$ under the hypothesis that sample $s$ belongs to stratum $k$ is:

$$\Pr(\mathbf{x}_s | s \in k) = \Pr\left[\chi_q^2 > d_s^k\right] . \tag{9}$$

The closer the sample is to the center of the stratum, the larger the tail probability, and $\Pr(\mathbf{x}_s | s \in k)$ can be used as a similarity measure of sample $s$ to stratum $k$. The distance $d_s^k$ is measured in the space spanned by the top $q$ eigenvectors, which captures the endogenous structure of the sample. Eq. (9) can be used in a Bayesian analysis to determine the probability that sample $s$ belongs to stratum $k$ given the variables $\mathbf{x}_s$, as described below. Other techniques for determining distance from a test sample to a stratum center can also be used.

At block 306, the predicted outcome for sample $s$ is computed based on the per-stratum predicted outcomes $y^k$ determined at block 302 and a per-stratum probability that sample s belongs to stratum $k$. (Note that training of the prediction model is assumed not to have included test sample $s$.) In some embodiments, a Bayesian model can be used to compute the prediction. For example, suppose that the prediction model has two outcomes: $y = 1$ corresponds to disease positive and $y = 0$ corresponds to disease negative. The predicted probability of disease for subject $s$ is the aggregated prediction from each stratum:

$$\Pr(y_s = 1 | \mathbf{x}_s) = \sum_{k=1}^{K} \Pr(y_s = 1 | s \in k, \mathbf{x}_s) \Pr(s \in k | \mathbf{x}_s) \tag{10}$$

where stratum-specific predictor Pr $(y_s = 1|s \in k, \boldsymbol{x}_s)$ is the result of the prediction model from that stratum. The probability Pr $(s \in k|\boldsymbol{x}_s)$ can be given by:

$$\Pr(s \in k|\mathbf{x}_s) = \frac{\Pr(s \in k; \mathbf{x}_s)}{\Pr(\mathbf{x}_s)} = \frac{\Pr(\mathbf{x}_s|s \in k)\Pr(s \in k)}{\sum_{k=1}^{K}\Pr(\mathbf{x}_s|s \in k)\Pr(s \in k)} \qquad (11)$$

where Pr$(s \in k) = n_k/N$ is the fraction of the training data set that is in stratum $k$, which is also the prior probability that a data sample belongs to stratum $k$. Pr$(\boldsymbol{x}_s|s \in k)$ is the probability of observing $\boldsymbol{x}_s$ when the data sample belongs to stratum $k$, e.g., as defined in Eq. (9).

Process 100 is illustrative, and variations and modifications are possible. For example, the number of data samples and variables can be chosen as desired. Any type of prediction model can be used, including but not limited to linear regression models. (It is assumed that the same type of prediction model and same set of input variables are used for all strata.) Process 100 is representative of a category of processes in which a training data set is stratified and prediction models are trained for different strata, and in which predictions for testing samples made by combining predictions from different strata according to the probability that the testing sample is in a particular stratum. Such processes are referred to herein as "Prism Vote" processes.

It is contemplated that a Prism Vote process such as process 100 can be performed by a computer and may operate on data sets of any size, having any number of variables. A set of prediction models and associated parameters (e.g., eigenvalues, eigenvectors, stratum centers) can be generated, e.g., by performing blocks 102-106, and the prediction models and parameters can be stored for later use. Obtaining testing samples and computing predicted outcomes for the testing sample (e.g., blocks 108 and 110) can be performed at any time after the prediction models have been trained and stored, and the same set of prediction models can be applied to any number of testing samples. In addition, the trained prediction models and associated parameters can be provided to computer systems other than the system that was used to train the prediction models. For example, a Prism Vote process for predicting disease can be trained by a research team, and the trained prediction models and associated parameters can be distributed to clinical workers (e.g., at a laboratory). The clinical workers can apply the trained prediction models and associated parameters to data collected from individual patients, e.g., by using a computer to compute a predicted outcome for a given patient. The predicted outcome can be used to inform treatment decisions or other care recommendations (e.g., changes in diet or lifestyle).

In some embodiments, a testing sample may be classified as an outlier if, for every stratum, its probability of belonging to that stratum (e.g., as determined from Eq. (11)) is less than a threshold (e.g., 0.05). Outliers can be treated differently from other testing samples. For instance, in addition to the per-stratum prediction models, a "global" prediction model can be trained on all of the training data samples (without stratification other segmentation), and the predicted outcome for an outlier can be determined using the global prediction model. Where a testing sample is identified as an outlier, any report of the predicted outcome can be annotated to indicate that the testing sample is an outlier.

**Performance of a Prism Vote Process**

A Prism Vote process such as process 100 allows different prediction models to be developed from different strata of a heterogeneous training data set. A tradeoff is that segmentation of the data set provides fewer data samples for training each per-stratum prediction model, which may mean each per-stratum prediction model may be less reliable. To understand when a Prism Vote process can be expected to provide more reliable predictions than a "global" prediction model (i.e., a single prediction model trained using all of the training data), one can consider the expected prediction error (EPE) for different approaches. For purposes of illustration, it is assumed that the prediction model is a linear regression model as described above. Similar analysis can be applied for other prediction models.

For a "global" linear regression model $\hat{f}_{LR}(x)$ trained on all training data samples, the EPE can be expressed as:

$$EPE_{LR}(x_0) = \mathrm{E}\left[\left(Y - \hat{f}_{LR}(x_0)\right)^2 |X = x_0\right]$$

$$= Var(Y|X = x_0) + \mathrm{E}[\hat{f}_{LR}(x_0) - \mathrm{E}\,\hat{f}_{LR}(x_0)]^2 \qquad (12)$$

$$= Var(Y|X = x_0) + Var\left(\hat{f}_{LR}(x_0)\right).$$

For a *K*-stratum Prism Vote process with a linear regression model trained for each stratum, the EPE can be expressed as:

$$EPE_{PV}(x_0) = \mathrm{E}\left[\left(Y - \hat{f}_{PV}(x_0)\right)^2 |X = x_0\right]$$

$$= E\left[\left(Y - \sum_{k=1}^{K} w_k(x_0)\hat{f}_k(x_0)\right)^2 |X = x_0\right]. \tag{13}$$

where $\hat{f}_k(x_0)$ is the prediction from the regression model of the kth stratum for test sample $x_0$ and $w_k(x_0)$ is the weight of the prediction for the *k*th stratum for test sample $x_0$.

Assuming a linear regression model, a Prism Vote process is expected to provide better accuracy than a global model when $EPE_{PV}(x_0) < EPE_{LR}(x_0)$. Derivation of the inequality reduces to *PVI* > 0, where the criterion PVI is given by:

$$PVI = \min_k \left\{\frac{\sigma^2}{\sigma_0^2} - \frac{[\frac{1}{\ln(n)} + x_0'(X^{k\prime}X^k)^{-1}x_0]}{[\frac{1}{\ln(Kn)} + x_0'(X'X)^{-1}x_0]}, k = 1,2,...,K\right\}, \tag{14}$$

where $\hat{\beta}^k = \begin{pmatrix} \hat{\beta}_0^k \\ \vdots \\ \hat{\beta}_p^k \end{pmatrix}$ is the least square estimates based on observations from stratum *k* according process 100,

$\hat{\beta} = \begin{pmatrix} \hat{\beta}_0 \\ \vdots \\ \hat{\beta}_p \end{pmatrix}$ is the least square estimates using the global linear regression model (trained on all samples), and

$$\sigma^2 = \sigma_0^2 + \frac{1}{[Kn - (p+1)]}\sum_{k=1}^{K}(\hat{\beta}^k - \hat{\beta})X^{k\prime}X^k(\hat{\beta}^k - \hat{\beta}). \tag{15}$$

When Eq. (14) yields *PVI* > 0, using a Prism Vote process with a linear regression model to train each stratum independently can be expected to provide better prediction performance than a single linear regression model trained on all the training samples.

In addition, when linear regression is used as the prediction model, the optimum number K of strata can be computed as:

$$\hat{K} = \arg\max_K \left\{\min_k \left[\frac{\sigma^2}{\sigma_0^2} - \frac{\left(\frac{1}{\ln(n)} + x_0'(X^{k\prime}X^k)^{-1}x_0\right)}{\left(\frac{1}{\ln(Kn)} + x_0'(X'X)^{-1}x_0\right)}\right], K = 1,2,...\right\}. \tag{16}$$

It should be understood that values of *K* other than that indicated by Eq. (16) may be selected, even if performance is sub-optimal. Further, where the prediction model is a model other than linear regression, similar logic can be used to define conditions under which a Prism Vote process is expected to outperform a single model and/or to determine an optimum number of strata.

**Example 1**

To illustrate selection of the number of strata, a simulation study of four data sets was performed. In each data set, a "true" number of strata was chosen (1, 2, 3, or 4), and data was generated assuming each stratum follows a different linear-regression prediction model. Prism Vote models were trained on each data set with different values of K (K = 1, 2, 3, 4, 5).

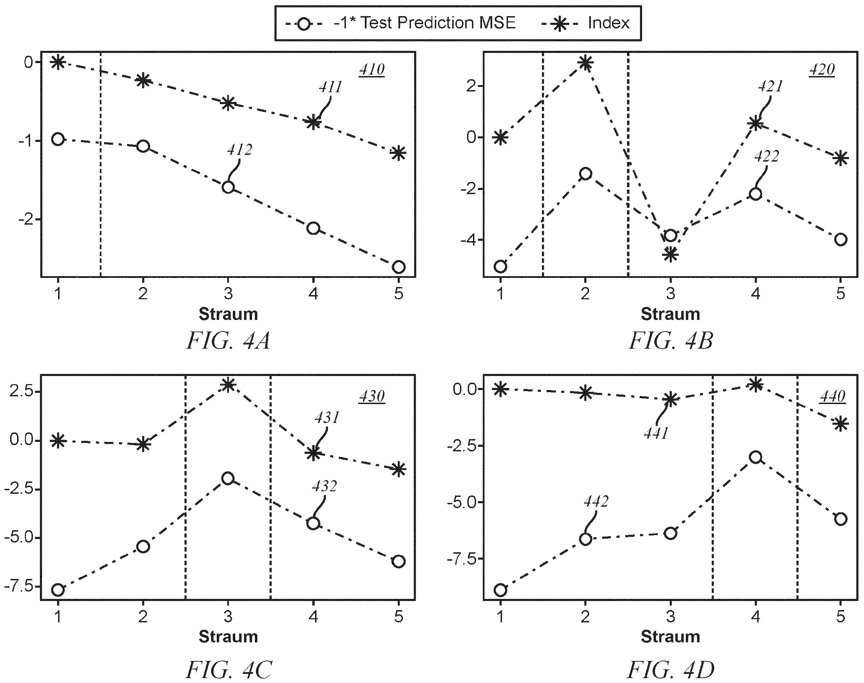

FIGs. 4A-4D show graphs 410, 420, 430, 440 corresponding to the four data sets. In Fig. 4A, the true number of strata was 1 (a traditional linear regression); in Fig. 4B, 2 strata; in Fig. 4C, 3 strata; in Fig. 4D, 4 strata. For each data set, PVI as defined by Eq. (14) above (lines 411, 421, 431, 441) and a mean square error (MSE) measure -1*MSE (lines 412, 422, 432, 442) are shown as a function of the number of strata K used in the Prism Vote process. In each case, PVI is maximized when K is chosen to be the true number of strata, and the K value that yields maximum PVI also provides the smallest MSE, as indicated by Eq. (16) above.

**Example 2**

To illustrate performance of a Prism Vote process, a simulation study has been performed using two populations having different phenotypes. Data was generated for five different scenarios. Each scenario used the same set of predictors (variables) and linear regression models, but different effect sizes (expressed as mean $\beta$ difference) for various predictors between the two populations. In Scenario 1, there was no difference between effect sizes (mean $\beta$ difference 0) for the two populations; in Scenarios 2-4, there were increasing degrees of difference between effect sizes (mean $\beta$ difference 0.18, 0.4, 0.67); and in Scenario 5, the effects were are completely different (mean $\beta$ difference 1). The resulting PVIs for Scenarios 1-5 were 0.27, 0.10, 0.76, 1.46 and 3.23. For each scenario, a mean squared error (MSE) was calculated for a global linear regression model (trained on all data samples) and for a Prism Vote process with $K = 2$.

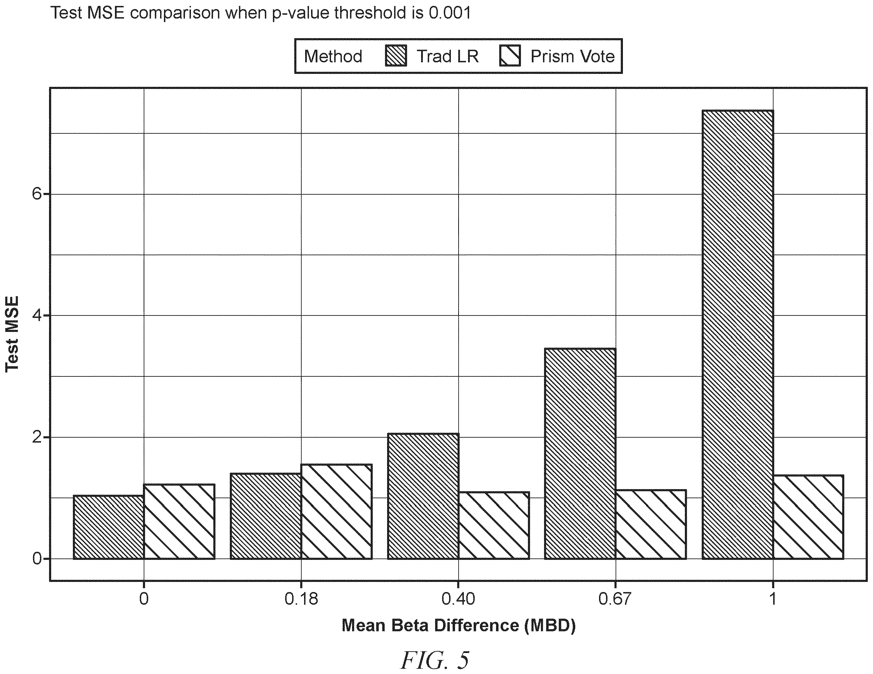

FIG. 5 is a bar chart comparing the MSE for the predictions in each scenario. For each scenario, the result for the global (traditional) linear regression model is on the left and the result for the Prism Vote process is on the right. It can be seen from FIG. 5 that when there is effect size difference, the PVI is greater than 0, and a Prism Vote process can provide a reduced MSE (better performance) compared to conventional processes.

**Example 3**

A Prism Vote process using a logistic-regression prediction model for each stratum has been applied to two whole-genome data sets associated with Alzheimer's disease and schizophrenia, respectively. A global logistic regression prediction model was also applied to the same data sets for comparison. Each trained model was applied to testing data for which the outcome was known, in order to assess sensitivity and specificity.

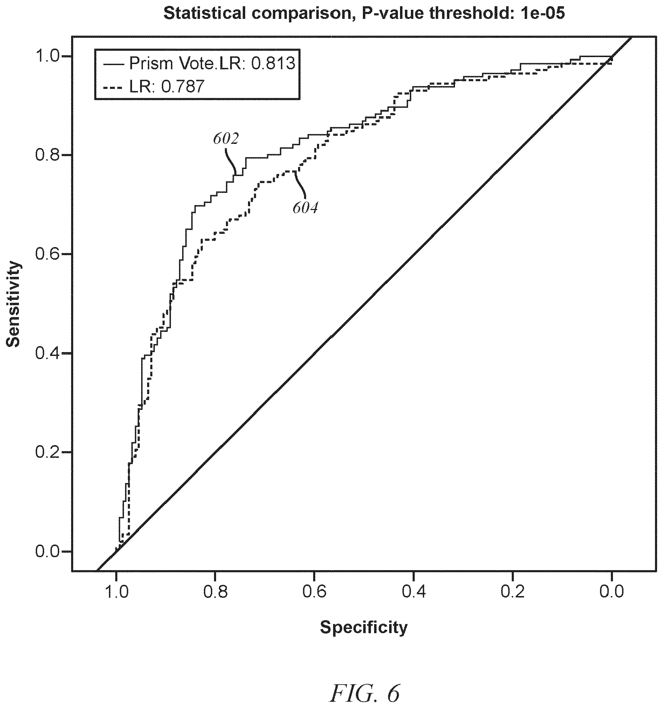

FIG. 6 is a graph showing receiver operating characteristic (ROC) curves for the Alzheimer's disease data for a Prism Vote process (line 602) and global logistic regression (line 604). With the Prism Vote process, the average Area-Under-the-Curve (AUC) reached 74.36% in 5-group cross validation (5GCV), making a 3.5% improvement over the conventional logistic regression.

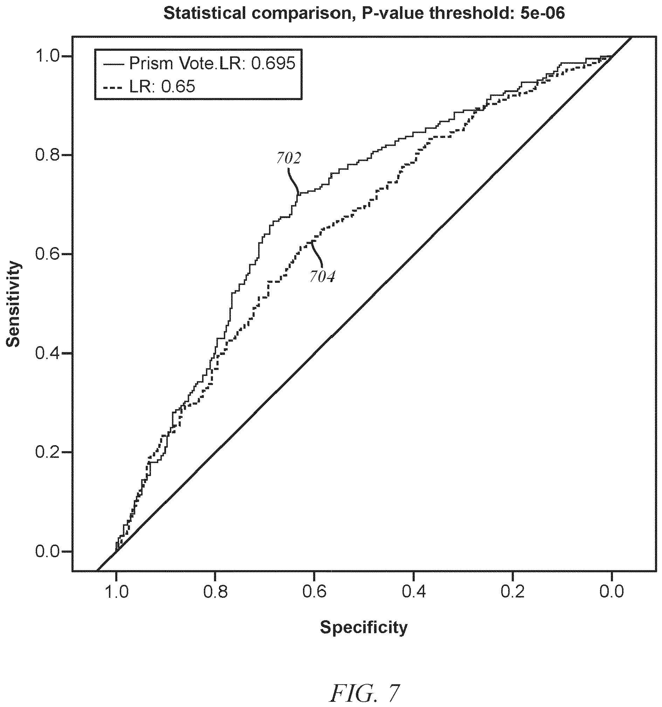

FIG. 7 is a graph showing ROC curves for the schizophrenia data for the Prism Vote process (line 702) and global logistic regression (line 704). With the Prism Vote process, the 5GCV average AUC is 68.2%, improved by 3.1% compared to the conventional logistic regression.

These examples show that accuracy of prediction can be improved using Prism Vote processes of the kind described herein. It should be understood that these examples are illustrative and not limiting. Performance may depend on the particular set of variables and outcomes being modeled, as well as on the prediction model used, the number of strata, and the size of the data set.

**Computer System Implementation**

Data analysis and computational operations of the kind described herein can be implemented in computer systems that may be of generally conventional design, such as a desktop computer, laptop computer, tablet computer, mobile device (e.g., smart phone), or the like. Such systems may include one or more processors to execute program code (e.g., general-purpose microprocessors usable as a central processing unit (CPU) and/or special-purpose processors such as graphics processors (GPUs) that may provide enhanced parallel-processing capability); memory and other storage devices to store program code and data; user input devices (e.g., keyboards, pointing devices such as a mouse or touchpad, microphones); user output devices (e.g., display devices, speakers, printers); combined input/output devices (e.g., touchscreen displays); signal input/output ports; network communication interfaces (e.g., wired network interfaces such as Ethernet interfaces and/or wireless network communication interfaces such as Wi-Fi); and so on. Computer programs incorporating various features of the claimed invention may be encoded and stored on various computer readable storage media; suitable media include magnetic disk or tape, optical storage media such as compact disk (CD) or DVD (digital versatile disk), flash memory, and other non-transitory media. (It should be understood that "storage" of data is distinct from propagation of data using transitory media such as carrier waves.) Computer readable media encoded with the program code may be packaged with a compatible computer system or other electronic device, or the program code may be provided separately from electronic devices (e.g., via Internet download or as a separately packaged computer-readable storage medium).

As described above, training of prediction models and application of trained prediction models to training data can be performed at different times and/or by different computer systems or the same computer system. Further, the training portion of a Prism Vote process can be repeated from time to time as additional training data becomes available.

**Additional Embodiments**

While the invention has been described with reference to specific embodiments, those skilled in the art will appreciate that variations and modifications are possible. All processes described above are illustrative and may be modified. Processing operations described as separate blocks may be combined, order of operations can be modified to the extent logic permits, processing operations described above can be altered or omitted, and additional processing operations not specifically described may be added. Particular definitions and data formats can be modified as desired.

In various embodiments, Prism Vote processes can be implemented using any type of prediction model and any number of strata, provided sufficient data is available to train the per-stratum prediction models. A Prism Vote process can stratify the data based on endogenous structure of the data set, e.g., as described above, and this may result in improved performance for a particular type of prediction model.

Further, while the foregoing examples make reference to disease prediction using genomic data, it should be understood that this is merely one use-case for a Prism Vote process. For example, genomic data may be used to predict any phenotypic characteristic, including disease (or absence thereof), response to treatment, expected physiological characteristics (e.g., blood sugar or cholesterol levels), effectiveness of preventive measures, and so on. Likewise, the input variables are not limited to genomic data. In a health-care context, any quantifiable information about an individual that may be correlated with a medical condition can be used as a variable. Examples include medical imaging data, blood test results, stress test results, and so on.

The applicability of Prism Vote processes is also not limited to the field of health care. For instance, in the financial sector, similar techniques can be used to predict performance of a publicly-traded stock based on a number of variables, where data sets may be heterogeneous in relation to factors such as industrial sector, dependence on weather or commodities, and so on.

Thus, although the invention has been described with respect to specific embodiments, it will be appreciated that the invention is intended to cover all modifications and equivalents within the scope of the following claims.

**Claims**

1. A method for predicting likelihood of an outcome based on a set of variables, the method comprising:

    identifying a training set of data samples, wherein for each data sample the variables and outcomes are known;
    segmenting the training set into a plurality of strata based on a measure of similarity of the data samples;
    training a prediction model for each stratum, wherein the prediction model predicts a likelihood of an outcome based on the variables and wherein training of the prediction model is performed independently for each stratum;
    obtaining a testing sample for which the variables are known; and
    predicting the outcome for the testing sample, wherein predicting the outcome includes:

        determining, for each stratum, a likelihood of the outcome using the prediction model for that stratum;
        determining, for each stratum, a probability that the testing sample belongs to that stratum; and
        computing a predicted outcome for the testing sample based on the likelihood for each stratum weighted by the probability that the testing sample belongs to that stratum.

2. The method of claim 1 wherein segmenting the training set includes:

    building a matrix for the training set of samples;
    computing a set of eigenvalues and a set of eigenvectors from the matrix;
    sorting the eigenvectors based on respective magnitudes of the eigenvalues; and
    using the sorted eigenvectors to segment the training set.

3. The method of claim 2 wherein using the sorted eigenvectors to segment the training set includes:

selecting a subset of the sorted eigenvectors as significant eigenvectors;
computing a weighted average vector of the significant eigenvectors, wherein the average is weighted according to the eigenvalues;
sorting components of the weighted average vector; and
using quantiles of the weighted average vector to assign each data sample from the training set to one of the strata.

4. The method of any preceding claim further comprising:
computing a center for each of the plurality of strata.

5. The method of claim 4 wherein determining, for each stratum, a probability that the testing sample belongs to that stratum includes computing a distance metric between the testing sample and the center of that stratum.

6. The method of any preceding claim wherein the predicted outcome for the testing sample is computed based on a Bayesian model.

7. The method of any preceding claim wherein the prediction model for each stratum is a linear regression model.

8. The method of any preceding claim wherein the prediction model for each stratum is a logistic regression model.

9. The method of any preceding claim wherein the variables include genomic information about a subject and the outcome corresponds to a health characteristic of the subject.

10. The method of claim 9 wherein the health characteristic is presence or absence of a disease.

11. A computer system comprising:

a memory; and
a processor coupled to the memory and configured to perform the method of any of claims 1 to 10.

*100*

| Identify training set of data samples | $\sim$ *102* |

$\downarrow$

| Segment training set into strata | $\sim$ *104* |

$\downarrow$

| Train prediction model independently for each stratum | $\sim$ *106* |

$\downarrow$

| Obtain testing sample | $\sim$ *108* |

$\downarrow$

| Compute prediction for testing sample based on prediction model for each stratum and probability that testing sample belongs to that stratum | $\sim$ *110* |

*FIG. 1*

*200*

Define matrix from training data — *202*

Compute eigenvalues and eigenvectors from matrix — *204*

Select top eigenvectors — *206*

Compute g vector — *208*

Use g vector to segment the training data into strata — *210*

Compute a center of each stratum — *212*

*FIG. 2*

*300*

For each stratum, determine prediction using prediction model for that stratum — *302*

For each stratum, determine probability that testing sample belongs to that stratum — *304*

Compute prediction for sample based on per-stratum prediction and per-stratum probability — *306*

*FIG. 3*

**FIG. 4A**

**FIG. 4B**

**FIG. 4C**

**FIG. 4D**

Test MSE comparison when p-value threshold is 0.001

*FIG. 5*

**Statistical comparison, P-value threshold: 1e-05**

*FIG. 6*

Statistical comparison, P-value threshold: 5e-06

FIG. 7

Europäisches
Patentamt

European
Patent Office

Office européen
des brevets

# EUROPEAN SEARCH REPORT

Application Number

EP 20 20 0864

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FAN Y.R. ET AL: "Development of PCA-based cluster quantile regression (PCA-CQR) framework for streamflow prediction: Application to the Xiangxi river watershed, China", APPLIED SOFT COMPUTING, vol. 51, 1 December 2016 (2016-12-01), pages 280-293, XP055780769, AMSTERDAM, NL ISSN: 1568-4946, DOI: 10.1016/j.asoc.2016.11.039 * abstract * * page 281 - page 287 * * page 289 - page 291; figure 2 * | 1-11 | INV. G06N20/20 |
| A | JA-WON SEO ET AL: "Novel PCA-based color-to-gray image conversion", 2013 IEEE INTERNATIONAL CONFERENCE ON IMAGE PROCESSING, IEEE, 15 September 2013 (2013-09-15), pages 2279-2283, XP032966035, DOI: 10.1109/ICIP.2013.6738470 [retrieved on 2014-02-11] * the whole document * | 1-11 | |
| A | Song Xiaoyu ET AL: "A New Estimating Equation Based Approach for Secondary Trait Analyses in Genetic Case-control Studies", Dr.P.H., Mailman School of Public Health, 27 June 2017 (2017-06-27), pages 1-95, XP055780566, Retrieved from the Internet: URL:https://academiccommons.columbia.edu/doi/10.7916/D8T15DB5 [retrieved on 2021-03-01] * the whole document * -/-- | 1-11 | |

TECHNICAL FIELDS
SEARCHED (IPC)

G06N

The present search report has been drawn up for all claims

| Place of search | Date of completion of the search | Examiner |
|---|---|---|
| The Hague | 2 March 2021 | Tsakonas, Athanasios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 20 0864

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CAO XUEYUAN ET AL: "POST: A framework for set-based association analysis in high-dimensional data", 2017 IEEE INTERNATIONAL CONFERENCE ON BIOINFORMATICS AND BIOMEDICINE (BIBM), IEEE, 13 November 2017 (2017-11-13), pages 332-338, XP033278328, DOI: 10.1109/BIBM.2017.8217672 [retrieved on 2017-12-15] * the whole document * | 1-11 | |
| A | HERSBACH HANS: "Decomposition of the Continuous Ranked Probability Score for Ensemble Prediction Systems", WEATHER AND FORECASTING, vol. 15, no. 5, 1 October 2000 (2000-10-01), pages 559-570, XP055780872, US ISSN: 0882-8156, DOI: 10.1175/1520-0434(2000)015<0559:DOTCRP>2.0.CO;2 * the whole document * | 1-11 | |

TECHNICAL FIELDS
SEARCHED (IPC)

The present search report has been drawn up for all claims

| Place of search | Date of completion of the search | Examiner |
|---|---|---|
| The Hague | 2 March 2021 | Tsakonas, Athanasios |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

**Patent documents cited in the description**

- US 62915459